Europäisches Patentamt

European Patent Office

Office européen des brevets

(19)

(11) Publication number: **0 044 793**
**B1**

(12) **EUROPEAN PATENT SPECIFICATION**

(45) Date of publication of patent specification: **04.06.86**

(21) Application number: **81401172.2**

(22) Date of filing: **22.07.81**

(51) Int. Cl.⁴: **C 12 N 7/00, C 12 N 5/00** // **C12M3/00**

(54) High titer production of hepatitis A virus.

(30) Priority: **23.07.80 US 171621**

(43) Date of publication of application:
**27.01.82 Bulletin 82/04**

(45) Publication of the grant of the patent:
**04.06.86 Bulletin 86/23**

(84) Designated Contracting States:
**AT BE CH DE FR GB IT LI LU NL SE**

(56) References cited:
**EP-A-0 008 559**
**EP-A-0 028 567**
**US-A-3 821 087**
**US-A-3 997 396**

(73) Proprietor: **MERCK & CO. INC.**
**126, East Lincoln Avenue P.O. Box 2000**
**Rahway New Jersey 07065 (US)**

(72) Inventor: **Markus, Henry Z.**
**1517 Thornberry Road**
**Wyncote Pennsylvania 19095 (US)**
Inventor: **McAleer, William J.**
**717 Marietta Drive**
**Ambler Pennsylvania 19002 (US)**

(74) Representative: **Martin, Jean-Jacques et al**
**Cabinet REGIMBEAU 26, Avenue Kléber**
**F-75116 Paris (FR)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Description

Hepatisis A is a liver disease which, while not commonly fatal, can involve many weeks of debilitating illness. It is usually spread by direct contact with an infected individual or by contaminated drinking water or food. Recent discoveries have enabled the *in vitro* propagation of this virus. Titers of the *in vitro* propagated virus, however, are usually low, i.e., about 2 or less as determined by the immune adherence hemagglutination assay (IAHA).

## Objects of the invention

It is an object of the present invention to provide an improved method for preparing HAV. Another object is to provide an *in vitro* tissue culture method for preparing HAV in high titer. These and other objects of the present invention will be apparent from the following description.

## Summary of the invention

HAV is produced in high titer from tissue cultures grown on a hollow fiber capillary. The growth cycle preferably contains two different elevated temperature stages with caffeine being present in the second stage.

## Detailed description

The present invention relates to a method of growing HAV *in vitro* and, more particularly, to a method of growing HAV *in vitro* in high titer from tissue culture grown on a hollow fiber capillary unit.

It has been found according to the present invention that higher yields of HAV are obtained when the tissue culture is grown on a hollow fiber capillary unit. The yield of HAV may be increased by using a growth cycle having two different temperature ranges, each temperature range being above room temperature. The second temperature range is lower than the first. A purine preferably is present during the second, lower temperature range. The first temperature range is from about 35 to about 38°C while the second temperature range is from about 30 to about 34°C. Preferably the first temperature is about 37°C and the second temperature is about 32°C. The purine is present in the *in vitro* cell culture nutrient medium in an amount effective to improve yield of HAV. The purine may be present in a level at which it exerts a detectable improvement in yield up to a level below which it exerts a toxic effect. Typically, the purine is employed at from about 0.0001 M to about 0.0007 M, preferably at from about 0.0001 M to about 0.0003 M. The purines include a number of products which are obtained by the hydrolysis of the nucleic acids found in the nucleoproteins of plant and animal cells. The purines are condensed ring systems comprised of the pyrimidine ring and the imidazole ring. Some suitable purines are, for example, caffeine, xanthine, theophylline, theobromine, paraxanthine, hypoxanthine, adenine, purine and guanine. Caffeine is a preferred purine.

The HAV may be grown in any type of cell culture system in which the virus is capable of replicating. The cell culture may be formed of primary, continuously cultivated, or transformed cells derived from kidney or liver of human or non-human primate origin. Specific examples of suitable cell cultures are those derived from fetal or newborn kidney cells from rhesus monkey (FRhK6), cynomolgus monkey, marmoset or cercopithecus monkey, or diploid fibroblast cells derived from human or non-human primate lung tissue such as, for example, WI-38 or MRC-5 cell lines. The cells are grown *in vitro* in the presence of a nutrient medium. By a nutrient medium is meant one which permits growth of cells *in vitro*. Some specific nutrient media are, for example, Medium 199, Morgan et al., *Proc. Soc. Exp. Bio. & Med.*, 73:1—8, 1950; Basal Medium Eagle, *Eagle Science, 122*, 501—504, 1955; Morton, *In Vitro*, Vol. 6, No. 2, pp. 89—108, 1970; Dulbecco's Modified Eagle's Medium, Dulbecco et al., *Virology, 8,* 396, 1959; Smith et al., *J. Virol., 12*, 185—196, 1960; Morton, *In Vitro*, Vol. 6, No. 2, pp. 89—108, 1970; Minimum Essential Medium (Eagle, *Science, 130*, 432 (1959) and RPMI Media, Moore et al., J.A.M.A. 199, 519—524 1967; Morton, *In Vitro* Vol. 6, No. 2, pp. 89—108, 1970.

The hollow fiber capillary unit is formed of a plurality of anisotropic hollow fiber membranes which provide a matrix for the culture of cells and organ explants. A bundle of these capillaries forms a three dimensional vascular system which permits controlled perfusion of cell aggregates with nutrients, as well as exchange of excreted substances. The capillaries consist of a sponge-like body with a very thin (0.5—1 µm), smooth layer of extremely fine, controlled pore size (approximate range: 0.001—0.01 µm) on the lumen side. From that surface outward, the pores become increasingly larger 5—10 µm at the perimeter. This structure provides a unique combination of selectively and extremely high permeability to liquids even at very low or zero pressure. The choice of desired membrane "cutoff" levels offers selective control of macromolecule transport. The open exterior of the capillaries presents a large surface for cell attachment and allows cells to penetrate toward the barrier at the lumen. The anisotropic fiber membranes may be prepared as described in U.S. patent 3,615,024. The bundle of hollow fiber capillary units may be prepared as described in U.S. patent 3,821,087.

The hollow fiber capillary functions not only as a permeable membrane, but also as an anisotropic base for cell growth. The hollow fiber capillary thus acts like a pseudoorgan.

While different types of cells have been grown on hollow fiber capillary units, e.g., mouse fibroblasts, human breast and choriocarcinoma, rat pancreas, rat pituitary tumor, rat villus crypt, human hepatocytes, rat hepatoma, monkey kidney, baby hamster ovary, and rat lung, due to the many variables in biologic materials, preparation and operating parameters, specific performance with other types of cells cannot be forecast.

The HAV obtained according to the present invention may be used in diagnostic assays for the detection of viral antibodies, and as a source of HAV for vaccine preparation.

The following examples illustrate the present invention without, however, limiting the same thereto.

Example 1

Following steam sterilization, a unit of artificial capillary bundles (Vitafiber[R] Amicon hollow-capillary unit 3S100) is connected to a reservoir bottle containing 1 liter of sterile nutrient medium (Medium 199 plus 1% fetal bovine serum and neomycin at a concentration of 50 µg/ml). A rhesus monkey kidney cell suspension (LLC-MK2) 2 ml, having a concentration of $1.65 \times 10^6$ cells/ml is inoculated through the side ports of the unit. The assembled unit is placed in a 37°C incubator with 5% $CO_2$. The nutrient medium is circualted through the capillary unit by a peristaltic pump at a rate of 5 ml/minute. The cells are grown for 16 days and then are infected with 0.5 ml of a HAV preparation having an IAHA titer of less than 1 or $10^7$—$10^8$ $ID_{50}$/ml. Cell growth is monitored by glucose utilization and viral titers are determined by immune adherence hemagglutination assay (IAHA). The following titers are obtained.

| Age of culture (days) | IAHA Titer |
| --- | --- |
| 7 | 0 |
| 14 | 0 |
| 27 | 8 |
| 34 | 32 |
| 37 | 4 |

Example 2

The procedure of Example 1 is repeated except that on day 44, the temperature is lowered to 32°C and $10^{-4}$ M caffeine is added to the nutrient medium. The following titers are obtained.

| Age of culture (days) | IAHA Titer |
| --- | --- |
| 7 | None |
| 14 | None |
| 27 | 8 |
| 34 | 32 |
| 36 | 4 |
| 37 | 4 |
| 44 | — |
| 47 | 8 |
| 55 | 8 |

It will be seen that the titers peaked at day 34 and declined rapidly thereafter but were boosted by lowering the temperature and adding caffeine.

**Claims**

1. A method for enhancing the yield of HAV in *in vitro* cell culture which comprises growing the virus in a susceptible cell culture planted on a hollow fiber capillary unit under conditions which permit viral replication.

2. A method according to claim 1 wherein the susceptible cell culture is allowed to attach to the hollow fiber capillaries before being infected by the HAV.

3. A method according to claim 1 wherein the cell culture is grown in two different elevated temperature stages with a first growth stage at an elevated temperature and with a second growth stage at a lower but still elevated temperature which is above room temperature with a purine optionally being present during the second growth stage in an amount effective to improve the yield of hepatitis A virus.

4. A method according to claim 1 wherein the first growth stage takes place at from 35°C to about 38°C.

5. A method according to claim 3 wherein the second growth stage takes place at from about 30°C to 34°C.

6. A method according to claim 1 wherein the second growth stage takes place at about 32°C.

7. A method according to claim 6 wherein the purine is present in an amount of from about 0.0001 M to about 0.0007 M.

8. A method according to claim 7 wherein the purine is present in an amount of from about 0.0001 M to about 0.0003 M.

9. A method according to claim 8 wherein the purine is caffeine.

## Patentansprüche

1. Ein Verfahren zur Erhöhung der Ausbeute von HAV in in-vitro-Zellkulturen, das das Züchten des Virus in einer suszeptiblen Zellkultur, die auf einer Hohlfaser-Kapillareneinheit unter Bedingungen gepflanzt ist, die virale Replikation erlauben, umfasst.

2. Verfahren nach Anspruch 1, bei dem man die suszeptible Zellkultur sich an den Hohlfaserkapillaren halten lässt, bevor sie durch das HAV infiziert wird.

3. Eine Methode nach Anspruch 1, bei der die Zellkultur in zwei verschiedenen Stufen von erhöhter Temperatur gezüchtet wird, mit einer ersten Wachstumsstufe bei einer erhöhten Temperatur und mit einer zweiten Wachstumsstufe bei einer niedrigeren, aber immer noch erhöhten Temperatur, die oberhalb Raumtemperatur liegt, wobei ein Purin gegebenenfalls während der zweiten Wachstumsstufe in einer Menge anwesend sein kann, die wirksam die Ausbeute von Hepatitis-A-Virus verbessert.

4. Methode nach Anspruch 3, bei der die erste Wachstumsstufe bei 35°C bis etwa 38°C erfolgt.

5. Eine Methode nach Anspruch 3, bei der die zweite Wachstumsstufe bei etwa 30°C bis 34°C erfolgt.

6. Eine Methode nach Anspruche 1, bei der die zweite Wachstumsstufe bei etwa 32°C erfolgt.

7. Eine Methode nach Anspruch 6, bei der das Purin in einer Menge von etwa 0,0001 M bis etwa 0,0007 M anwesend ist.

8. Methode nach Anspruch 7, bei der das Purin in einer Menge von etwa 0,0001 M bis etwa 0,0003 M anwesend ist.

9. Methode nach Anspruch 8, bei der das Purin Coffein ist.

## Revendications

1. Méthode pour accroître le rendement du virus de l'hépatite A dans une culture de cellules *in vitro* qui consiste à faire pousser le virus dans une culture cellulaire sensible ensemencée sur une unité capillaire à fibres creuses dans des conditions qui permettent le réplication du virus.

2. Méthode selon la revendication 1, caractérisée en ce qu'on laisse la culture cellulaire sensible se fixer aux capillaires à fibres creuses avant d'être infectée par le virus de l'hépatite A.

3. Méthode selon la revendication 1, caractérisée en ce qu'on fait croître la culture cellulaire en deux phases distinctes de température élevée avec une première phase de croissance à température élevée et avec une seconde phase de croissance à une température plus basse, mais toujours élevée, qui est supérieure à la température ambiante, une purine étant facultativement présente pendant le seconde phase de croissance à une teneur efficace pour améliorer le rendement du virus de l'hépatite A.

4. Méthode selon la revendication 3, caractérisée en ce que la première phase de croissance a lieu entre 35°C et environ 38°C.

5. Méthode selon la revendication 3, caractérisée en ce que la seconde phase de croissance a lieu entre environ 30°C et 34°C.

6. Méthode selon la revendication 1, caractérisée en ce que la seconde phase de croissance a lieu à environ 32°C.

7. Méthode selon la revendication 6, caractérisée en ce que le purine est présente à une teneur entre environ 0,0001 M et environ 0,0007 M.

8. Méthode selon la revendication 7, caractérisée en ce que la purine est présente à une teneur entre environ 0,0001 M et environ 0,0003 M.

9. Méthode selon la revendication 8, caractérisée en ce que la purine est la caféine.